# EUROPEAN PATENT APPLICATION

(11) **EP 4 732 750 A1**
(43) Date of publication of application: **29.04.2026**
(21) Application number: 23953377.1
(22) Date of filing: 26.09.2023
(51) Int. Cl.: A61B 1/04

(54) **ENDOSCOPE AND DIAPHRAGM OF ENDOSCOPE**

(71) Applicant: Shenzhen Mindray Bio-Medical Electronics Co., Ltd., Shenzhen, Guangdong 518057 (CN)
(72) Inventor: YUAN, Xiaowen, Shenzhen, Guangdong 518057 (CN); ZHU, Jie, Shenzhen, Guangdong 518057 (CN); WANG, Shaofei, Shenzhen, Guangdong 518057 (CN)
(74) Representative: KIPA AB
(86) International application number: PCT/CN2023/121385
(87) International publication number: WO 2025/065216

(57) **Abstract**

The present invention relates to an endoscope. Provided are an endoscope and a diaphragm of the endoscope. The endoscope comprises: a tube unit, which is configured to be inserted into a part to be observed; and an optical imaging system, which is arranged in the tube unit and comprises a diaphragm, wherein a through hole is formed in the radial middle portion of the diaphragm, and the through hole forms a first light-passing area; the diaphragm is further provided with a second light-passing area located on a radial outer side of the first light-passing area, and the second light-passing area is configured to absorb light within a first wavelength interval and allow light within a second wavelength interval to pass therethrough; and the light within the first wavelength interval is the light reflected after a light source of the endoscope irradiates the part to be observed, and the light within the second wavelength interval is fluorescence generated by the light source of the endoscope irradiating fluorescent dye at the part to be observed. The present invention mainly solves the technical problem of contradiction between the depth of field of visible light and the signal strength of fluorescence in the endoscope.

## Description

### TECHNICAL FIELD

The disclosure relates to a technical field of endoscope, and in particular, to an endoscope and a diaphragm of an endoscope.

### BACKGROUND

In common fluorescence navigation surgery, an endoscope needs to be able to simultaneously image a visible light (also known as a white light in industry, typically covering a wavelength range of approximately 400 to 700 nanometres) and fluorescence (usually in a near-infrared band, typically covering a wavelength range of approximately 700 to 1300 nanometres). A visible light can provide a doctor with a clear image within a range for a certain depth of field, and fluorescence can help a doctor to identify more deep tissue structures. Due to a deep penetration depth of an infrared light, in order to achieve fluorescence imaging, fluorescent dyes, such as ICG (indocyanine green) and methylene blue (also known as infrared excitation dyes), are currently selected as contrast agents, which can produce fluorescence in an infrared band when excited. However, a detectable signal strength of an infrared light is relatively weak.

A diaphragm, especially an aperture stop, is arranged inside the endoscope to limit a light beam. The aperture stop can limit an amount of a light that enters an optical system, and a size of the aperture stop also determines an intensity of a light signal and an effect for a depth of field. The larger an aperture stop, the stronger an intensity of a fluorescence signal, but the worse a depth of field for a visible light, that is, a signal intensity and a depth of field are contradictory to each other.

Therefore, how to maximize an intensity and a sensitivity of a fluorescence signal without affecting a depth of field for a visible light image is a major pain point in a field of endoscopy.

### SUMMARY

### Technical problem

This disclosure mainly solves a technical problem of a contradiction between a depth of field for a visible light and a signal intensity for fluorescence in an endoscope.

### Technical solution

In a first aspect, this disclosure provides an endoscope.

An endoscope, including:
a tube unit, which is inserted into a portion to be observed; and
an optical imaging system, which is arranged inside the tube unit, wherein the optical imaging system includes a diaphragm;
wherein the diaphragm is provided with a through-hole at a middle of the diaphragm along a radial direction of the diaphragm, wherein the through-hole forms a first light-transmitting region; the diaphragm is further provided with a second light-transmitting region, which is located outside the first light-transmitting region along a radial direction of the first light-transmitting region; wherein the second light-transmitting region is configured to absorb a light within a first wavelength range and transmit a light within a second wavelength range; wherein the light within the first wavelength range is a light that is reflected after a light source of the endoscope irradiates the portion to be observed, and the light within the second wavelength range is fluorescence that is generated by a fluorescent dye at the portion to be observed when the light source of the endoscope irradiates the fluorescent dye.

In a technical solution, the diaphragm is arranged at a position of an aperture stop of the optical imaging system, and/or at conjugate position(s) of object and image of an aperture stop of the optical imaging system.

In a technical solution, the endoscope is a rigid endoscope, and the endoscope includes a rod lens assembly for image inversion, wherein the rod lens assembly includes a pair of rod lenses, and the diaphragm is arranged between the pair of rod lenses.

In a technical solution, at least a portion of respective end surfaces of respective adjacent ends of the pair of rod lenses is a convex spherical surface; at least a portion of the convex spherical surface is inserted into the through-hole at the middle of the diaphragm.

In a technical solution, the endoscope is a flexible endoscope or an electronic endoscope, the diaphragm is located on an objective lens of the flexible endoscope or the electronic endoscope.

In a second aspect, this disclosure provides another endoscope.

An endoscope, including:
a tube unit, which is inserted into a portion to be observed; and
an optical imaging system, which is arranged inside the tube unit, wherein the optical imaging system includes a diaphragm;
wherein the diaphragm is provided with a first light-transmitting region, which is located at a middle of the diaphragm along a radial direction of the diaphragm, and a second light-transmitting region, which is located outside the first light-transmitting region along a radial direction of the first light-transmitting region; wherein the first light-transmitting region is configured to transmit a light within a first wavelength range and a light within a second wavelength range; the second light-transmitting region is configured to absorb the light within the first wavelength range and transmit the light within the second wavelength range; wherein the light within the first wavelength range is a light that is reflected after a light source of the endoscope irradiates the portion to be observed, and the light within the second wavelength range is fluorescence that is generated by a fluorescent dye at the portion to be observed when the light source of the endoscope irradiates the fluorescent dye.

In a third aspect, this disclosure provides another endoscope.

An endoscope, including:
a tube unit, which is inserted into a portion to be observed; and
an optical imaging system, which is arranged inside the tube unit, wherein the optical imaging system includes a diaphragm;
wherein the diaphragm is provided with a through-hole at a middle of the diaphragm along a radial direction of the diaphragm, wherein the through-hole forms a first light-transmitting region; the diaphragm is further provided with a second light-transmitting region, which is located outside the first light-transmitting region along a radial direction of the first light-transmitting region; wherein the second light-transmitting region is configured to absorb a light within a first wavelength range and transmit a light within a second wavelength range; wherein a wavelength value of the first wavelength range is less than a wavelength value of the second wavelength range.

In a technical solution, a physical thickness d of the diaphragm is configured to be: d>0.3 mm.

In a technical solution, the light within the first wavelength range is a visible light, and a transmittance for the visible light of the second light-transmitting region is no more than 1%.

In a technical solution, the light within the second wavelength range is a near-infrared light, and a transmittance for the near-infrared light of the second light-transmitting region is not less than 50%.

In a technical solution, an outer diameter of the second light-transmitting region is greater than 1.2 times of an outer diameter of the first light-transmitting region.

In a technical solution, the first light-transmitting region is made of a homogeneous material, and/or the second light-transmitting region is made of a homogeneous material.

In a technical solution, a material of the first light-transmitting region is one of: acrylonitrile-butadiene-styrene copolymer, polymethyl methacrylate, polyethylene terephthalate plastic, polyetherimide, and polycarbonate; and/or
a material of the second light-transmitting region is one of: acrylonitrile-butadiene-styrene copolymer, polymethyl methacrylate, polyethylene terephthalate plastic, polyetherimide, and polycarbonate.

In a technical solution, a high-temperature resistance of the diaphragm is not less than 110 degrees Celsius.

In a technical solution, the diaphragm is provided with a third light-transmitting region, which is located between the first light-transmitting region and the second light-transmitting region, and/or is located outside the second light-transmitting region along a radial direction of the second light-transmitting region.

In a fourth aspect, this disclosure provides a diaphragm for an optical imaging system of an endoscope.

A diaphragm for an optical imaging system of an endoscope, including:
a first light-transmitting region, which is located at a middle of the diaphragm along a radial direction of the diaphragm; and
a second light-transmitting region, which is located outside the first light-transmitting region along a radial direction of the first light-transmitting region;
wherein the first light-transmitting region is configured to transmit a light within a first wavelength range and a light within a second wavelength range; the second light-transmitting region is configured to absorb the light within the first wavelength range and transmit the light within the second wavelength range; wherein the light within the first wavelength range is a light that is reflected after a light source of the endoscope irradiates the portion to be observed, and the light within the second wavelength range is fluorescence that is generated by a fluorescent dye at the portion to be observed when the light source of the endoscope irradiates the fluorescent dye; wherein a wavelength value of the first wavelength range is less than a wavelength value of the second wavelength range.

In a technical solution, the diaphragm is provided with a through-hole at the middle of the diaphragm along the radial direction of the diaphragm, wherein the through-hole forms the first light-transmitting region.

### Beneficial effect

Beneficial effects of this disclosure are as follows.

According to the above-mentioned endoscope, a first light-transmitting region (which can be a through-hole) is located at a middle of a diaphragm along a radial direction of the diaphragm, a second light-transmitting region is located outside the first light-transmitting region along a radial direction of the first light-transmitting region, the second light-transmitting region is configured to absorb a light within a first wavelength range and transmit a light within a second wavelength range with a wavelength value higher than that in the first wavelength range, which is equivalent to that, a smaller diaphragm for a light with a relatively small wavelength value (such as a light that is reflected after a light source of an endoscope irradiates a portion to be observed) is arranged, so as to obtain a large depth of field, and meanwhile a larger diaphragm for a light with a relatively large wavelength value (such as fluorescence that is generated by a fluorescent dye at the portion to be observed when a light source of an endoscope irradiates the fluorescent dye), is arranged, so as to obtain a stronger signal intensity, thereby solving a technical problem of contradiction between a depth of field for a visible light and a signal intensity for fluorescence in an endoscope. Meanwhile, the second light-transmitting region employs light absorption to prevent a light within the first wavelength range from transmitting without requiring a coating solution, such that a simpler process can be adopted, which is easy to process and has a lower cost, and no problem of oxidation of coating exists, thus obtaining a better stability.

### BRIEF DESCRIPTION OF THE DRAWINGS

FIG. 1 is a diagram of an optical system in an endoscope according to an embodiment.
FIG. 2 is a partially enlarged view of point A in FIG. 1.
FIG. 3 is a diagram of a working principle of a diaphragm of an endoscope of this disclosure.
FIG. 4 is a curve graph for a transmittance for a light of an aperture stop in FIG. 1.
FIG. 5 is an experimental comparison diagram for ICG fluorescence of an aperture stop in FIG. 1; in which left and right experimental graphs take the aperture stop as a single variable.
FIG. 6 is a longitudinal sectional view of a diaphragm of an endoscope of this disclosure.

List of feature names corresponding to reference numerals in drawings are as follows.
100, aperture stop; 110, first light-transmitting region; 120, second light-transmitting region;
210, objective lens unit; 220, image inversion unit; 221, rod lens assembly; 222, rod lens; 223, convex spherical surface; 230, eyepiece unit.

### DESCRIPTION OF THE PREFERRED EMBODIMENTS

This disclosure will be further described in detail below through specific embodiments with reference to the accompanying drawings. Similar elements in different embodiments are labeled with similar element numbers. In the following embodiments, many details are described to enable a better understanding of this disclosure. However, those skilled in the art will readily recognize that some of the features may be omitted in different situations, or may be replaced by other elements, materials, or methods. In some cases, some operations related to this disclosure are not shown or described in the description. This is to avoid the core part of this disclosure being overwhelmed by excessive description. For those skilled in the art, it is not necessary to describe these related operations in detail. They can fully understand the related operations based on the description in the description and the general technical knowledge in this field.

In addition, features, operations, or characteristics described in the description may be combined in any appropriate manner to form various embodiments. At the same time, steps or actions in the method description may also be interchanged or adjusted in a manner that is obvious to those skilled in the art. Therefore, the various orders in the description and the drawings are only for the purpose of clearly describing a certain embodiment and are not meant to be a necessary order, unless otherwise specified that a certain order must be followed.

Serial numbers assigned to components in this document, such as "first", "second", etc., are only configured to distinguish objects described and do not have any order or technical meaning. Unless otherwise specified, terms "connection" and "coupling" mentioned in this disclosure include direct and indirect connections (couplings).

Diaphragm refers to an entity that limits a light beam in an optical system, and can be an edge of a lens, a frame, or a specially designed screen with a hole. In an optical system, a diaphragm can limit a light beam or restrict a size of a field of view (an imaging range). An aperture stop is a type of a diaphragm exists in all optical systems. If a diaphragm or an image of a diaphragm is looked from an object point, one with a smallest field angle determines an aperture stop of an optical system. The aperture stop can limit an amount of light entering into the optical system. In short, an aperture stop is like a window, which controls an amount of light entering into the optical system by adjusting its size. A size of an aperture stop affects a brightness and a depth of field of imaging. When the aperture stop is larger, more light can enter into the optical system, imaging will be brighter, and a depth of field will be shallower (that is, respective focus ranges of a foreground and a background will be smaller). When the aperture stop is smaller, less light enters into the optical system, imaging will be darker, and a depth of field will be deeper (that is, respective focus ranges of a foreground and a background will be larger).

During endoscopic imaging, lights at a portion to be observed include a light that is reflected after a light source of the endoscope irradiates the portion to be observed, as well as fluorescence that is generated by a fluorescent dye at the portion to be observed when a laser irradiates the fluorescent dye. As the reflected light and the fluorescence generated by excitation at the portion to be observed both need to pass through the optical system of the endoscope, a design of the optical system needs to consider how to achieve ideal imaging of both types of lights. In particular, the optical system of the endoscope needs to consider how to enable fluorescence with a weaker strength to have a stronger signal intensity, while enabling a white light to have a larger depth of field, so as to obtain an ideal image quality.

An embodiment of an endoscope in this disclosure provides a rigid tube endoscope (also called a rigid endoscope, a hard endoscope) including an aperture stop with a high transmittance for fluorescence, wherein the aperture stop 100 included therein is made of a material having a high absorption property for a white light and a high transmittance property for an infrared right. Since the diaphragm is located at a position of an aperture stop of the entire system, a strong absorption property of the diaphragm for a white light limits a beam diameter of the white light, ensuring an effect of a depth of field for a white light image; while a high transmittance of the diaphragm for an infrared light enables a beam diameter of the infrared light transmitted to be larger than a beam diameter of the white light transmitted, ensuring a signal strength for an infrared light. In this way, a purpose of greatly improving a fluorescence sensitivity during surgery, without affecting a depth of field for a white light image of the optical rigid tube endoscope, can be obtained. At the same time, this solution utilizes an absorption feature of material itself. Compared with other methods that use a reflective coating to reduce a size of an aperture stop for a white light, this solution not only avoids a problem of affecting contrast by stray light formed inside the system after a reflection of the white light, but also has many advantages compared to a solution of making a diaphragm of reflective coating on a bonding surface of a cemented lens, such as simpler processing, higher stability, and lower cost.

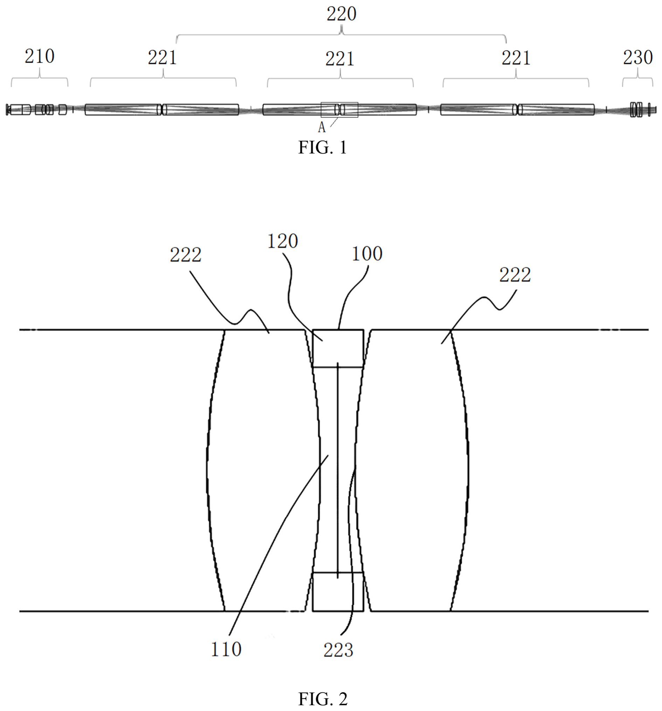

In an embodiment, a rigid tube endoscope includes a tube body unit and an optical imaging system. The tube unit is inserted into a portion to be observed and is capable of providing a corresponding arrangement space for the optical imaging system. Referring FIG. 1, the optical imaging system may include an objective lens unit 210, an image inversion unit 220, and an eyepiece unit 230. The objective lens unit 210, the image inversion unit 220, and the eyepiece unit 230 may adopt existing architectures and will not be described in detail here. However, the aperture stop 100 in the optical imaging system of this disclosure adopts a special structure.

Please refer to FIG. 2, a through-hole is provided at a middle of the aperture stop 100 along a radial direction of the aperture stop 100 of the optical imaging system, wherein the through-hole forms a first light-transmitting region 110. The aperture stop 100 is further provided with a second light-transmitting region 120, which is located outside the first light-transmitting region along a radial direction of the first light-transmitting region 110, wherein the second light-transmitting region 120 is configured to absorb a light within a first wavelength range, and transmit a light within the second wavelength range. The light within the first wavelength range is a light that is reflected after a light source of the endoscope irradiates the portion to be observed, and the light within the second wavelength range is fluorescence that is generated by a fluorescent dye at the portion to be observed when the light source of the endoscope irradiates the fluorescent dye. Those skilled in the art should understand that the above-mentioned radial direction refers to a direction perpendicular to an optical axis of a diaphragm (the above mentioned aperture stop 100), and the middle of the diaphragm along the radial direction of the diaphragm is a region with a certain radial size centered on an optical axis of the aperture, which region is relative to a second light-transmitting region 120 on an outer periphery, and its range can be large or small.

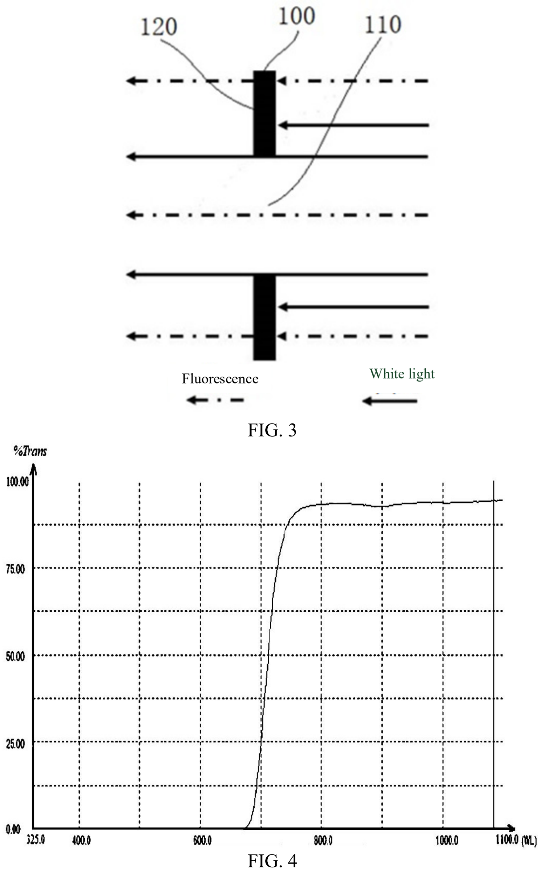

In addition, it should be noted that, for an optical imaging system, an optical element absorbing a light means that, the optical element absorbs a designated light with a purpose of preventing the designated light from passing through, for example, an absorption rate greater than 50%. Similarly, an optical element transmitting a light generally means that, the optical element transmits a designated light with a purpose of allowing the designated light to pass through, for example, a transmittance greater than 50%. Specifically, for the second light-transmitting region 120 of the aperture stop 100, "absorbing" in this disclosure may refer to an absorption rate for a designated light of not less than 90%, and "transmitting" may refer to a transmittance for a designated light of not less than 90%, which can better satisfy an optical performance requirement. Preferably, the light within the first wavelength range is a visible light that is reflected after a light source of an endoscope irradiates the portion to be observed, and a transmittance for the visible light of the second light-transmitting region 120 is not more than 1%, and the light within the second wavelength range is an near-infrared light that is generated by a fluorescent dye at the portion to be observed when the light source of the endoscope irradiates the portion to be observed, and a transmittance for the near-infrared light of the second light-transmitting region 120 is not less than 50%. Of course, in other embodiments, the above-mentioned absorption rate and transmittance may also be higher or lower, but "absorbing" refers to mainly absorbing a designated light, and "transmitting" refers to mainly transmitting a designated light. This can still play a role in improving optical performance to a certain extent, that is, it can enhance an signal intensity of fluorescence and reduce an impact on a depth of field of a white light. Of course, since different fluorescent dyes may generate fluorescence of different wavelengths, a corresponding light-transmitting region of the aperture stop should satisfy ""transmitting" characteristics of fluorescence of corresponding wavelength(s). For example, an infrared ray include a near infrared light and a far infrared light, and for an endoscope, a near infrared light is generally selected as fluorescence. In a specific embodiment, a light transmittance curve of the diaphragm can refer to FIG. 4.

In some embodiments, a diaphragm using the above structure can theoretically be arranged at a position, which is not a position of an aperture stop of the optical imaging system. However, arranging said diaphragm at a position of an aperture stop of the optical imaging system, and/or at conjugate position(s) of object and image of an aperture stop of the optical imaging system, can ensure a best effect. Of course, considering a cost issue, the diaphragm can generally be arranged only at a position of an aperture stop of the optical imaging system, which is, using the diaphragm as the aperture stop, or only at conjugate position(s) of object and image of an aperture stop of the optical imaging system. Those skilled in the art will understand that conjugate position(s) of object and image of an aperture stop refers to other imaging position(s) of an aperture stop through internal optical element(s) in the optical system.

In one embodiment, for a rigid tube endoscope, please refer to FIG. 1, the endoscope includes multiple groups of rod lens assemblies 221 for image inversion, a rod lens assembly 221 includes a pair of rod lenses 222, and the aperture stop 100 is arranged between the pair of rod lenses 222. Since an aperture stop 100 of a rigid tube endoscope is usually disposed between a pair of rod lenses 222, this method is adopted in this disclosure to facilitate optical path design without requiring many adjustments to the existing optical structure. Specifically, in one embodiment, referring to FIG. 2, respective end surfaces of respective adjacent ends of the pair of rod lenses 222 are convex spherical surfaces 223, and at least a portion of a convex spherical surface 223 is inserted into the through-hole at the middle of the aperture stop 100. This structure is advantageous for achieving centering of the aperture stop 100 and the rod lens 222. Of course, in other embodiments, respective end surfaces of respective adjacent ends of the pair of rod lenses 222 may not be convex spherical surfaces 223. In this case, the aperture stop 100 can achieve radial positioning by relying on another structure, such as relying on the tube unit to achieve radial positioning. In addition, in another embodiment, a gap may exist between the aperture stop 100 and the rod lens 222, which does not affect the function of the aperture stop 100 in this disclosure.

It should be noted that, in other embodiments, the aperture stop 100 of the rigid tube endoscope may also be arranged outside the rod lens assembly 221. In addition, an endoscope can generally be classified into a rigid tube endoscope, a flexible endoscope (also called as a flexible tube endoscope) and an electronic endoscope (also called electronic tube endoscope). In some other embodiments, the diaphragm with a high transmittance for fluorescence in this disclosure can also be located at an objective lens of the flexible endoscope or the electronic endoscope. Different from a rigid tube endoscope, an internal structure of an electronic endoscope can be a circuit system, the eyepiece is a micro camera chip, a signal wire exists inside the tube unit. The electronic endoscope collects image signals through an eyepiece camera chip, and transmits signals through wires inside the tube. The entire electronic endoscope transmits electrical signals, rather than light signals. The flexible endoscope using optic fiber also uses an objective lens to form an image, but generally uses optical fibers to transmit light, this is generally low in cost and has a limited resolution.

In a specific embodiment, the first light-transmitting region 110 is formed by a through-hole, which can be considered as having a transmittance of 100% for both a white light and fluorescence, while the second light-transmitting region 120 is made of a homogeneous material. For example, a material of the second light-transmitting region 120 can be one of acrylonitrile-butadiene-styrene copolymer, polymethyl methacrylate, polyethylene terephthalate plastic, polyetherimide, and polycarbonate. In addition to the aforementioned plastic material, the second light-transmitting region 120 may also be made of glass. It should be noted that the homogeneous material is not limited to a material having a single component. For example, a homogeneous material having uniform dopants can be formed by uniformly doping other substance(s) other than a base material.

In one embodiment, a physical thickness d of the aperture stop 100 is configured to be: d>0.3 mm, which is beneficial to ensuring an absorption effect of white light, preventing light leakage, and ensuring its own structural strength.

In order to achieve a better optical performance, in one embodiment, an outer diameter of the second light-transmitting region 120 is greater than 1.2 times of an outer diameter of the first light-transmitting region 110.

For example, for the above-mentioned diaphragm with a high transmittance for fluorescence, an equivalent size of an aperture stop for a white light may be 4.9 mm, and an equivalent size of an aperture stop for fluorescence may be 6.1 mm. Of course, an outer diameter of each light-transmitting region can be arranged according to an imaging requirement of the optical system. The outer diameter of the second light-transmitting region 120 is greater than 1.2 times of the outer diameter of the first light-transmitting region 110, which is conducive to both white light and fluorescence having excellent image quality close to a diffraction limit.

Reusable rigid tube endoscope needs to be sterilized after use, for example, by being sterilized at a high temperature exceeding 100 degrees Celsius.

In order to satisfy the above-mentioned disinfection requirement, in an embodiment, a high-temperature resistance of the diaphragm is not less than 110 degrees Celsius. Of course, when there is no need for high-temperature disinfection, the above-mentioned high-temperature resistance performance may not be considered.

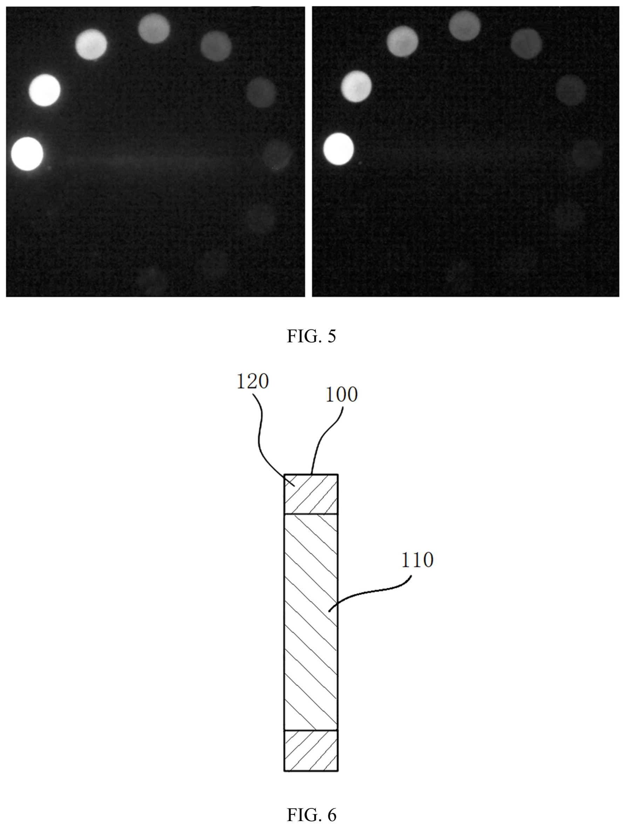

In the above embodiment, a through-hole is provided at a middle of a diaphragm with a high transmittance for fluorescence along a radial direction of said diaphragm, and said through-hole forms the first light-transmitting region 110. However, in some embodiments, please refer to FIG. 6, the diaphragm with a high transmittance for fluorescence can also be a solid structure. For example, the first light-transmitting region 110 is made of a material that transmits a white light and fluorescence at the same time. Optionally, a material of the first light-transmitting region 110 can also be a homogeneous material, for example, one of acrylonitrile-butadiene-styrene copolymer, polymethyl methacrylate, polyethylene terephthalate plastic, polyetherimide, and polycarbonate, and a corresponding performance can also be achieved by doping corresponding substance(s). In addition to the aforementioned plastic material, the first light-transmitting region 110 may also be made of glass. Similar to the second light-transmitting region 120, for the first light-transmitting region 110, the first light-transmitting region 110 transmits a light within the first wavelength range and a light within the second wavelength range. "Transmitting" means mainly allowing a designated light to pass through. Preferably, "transmitting" can mean that a transmittance of a designated light is not less than 90%, which can better satisfy an optical performance requirement.

Of course, in some other embodiments, the first light-transmitting region 110 and/or the second light-transmitting region 120 of the diaphragm with a high transmittance for fluorescence can also be made of non-homogeneous material(s), for example, being arranged as a multi-layer structure along the axial direction of the diaphragm, or as a material which is gradually changed along a radial direction. In addition, this disclosure does not exclude a scheme of arranging a light-transmitting region other than the first light-transmitting region 110 and the second light-transmitting region 120 on the diaphragm. For example, in a specific embodiment, the diaphragm with a high transmittance for fluorescence may include a third light-transmitting region, which is located between the first light-transmitting region 110 and the second light-transmitting region 120. In some other embodiments, the third light-transmitting region may also be located outside the second light-transmitting region 120 along a radical direction of the second light-transmitting region 120, and the diaphragm may also have more light-transmitting regions to achieve corresponding purposes.

In the above embodiment, the light within the first wavelength range is the light that is reflected after the light source of the endoscope irradiates the portion to be observed, and the light within the second wavelength range is the fluorescence that is generated by the fluorescent dye at the portion to be observed when the light source of the endoscope irradiates the fluorescent dye. In some other embodiments, the above-mentioned diaphragm with a high transmittance for fluorescence can also be used in other scenarios. As long as a wavelength value of the first wavelength range is less than a wavelength value of the second wavelength range, the light within the first wavelength range can correspond to a smaller diaphragm size, and the light within the second wavelength range can correspond to a smaller diaphragm size.

An embodiment of a diaphragm for an endoscope optical imaging system in this disclosure has a structure that is the same as the aperture stop 100 in any of the above-mentioned embodiments of the endoscope, and will not be described in detail here.

In this disclosure, for an endoscope, by arranging the above-mentioned diaphragm with a high transmittance for fluorescence, a beam diameter of a white light can be limited by a strong absorption characteristic of the diaphragm for the white light, thus ensuring an effect of a depth of field of a white light image; and meanwhile a beam diameter of a transmitted infrared beam larger is made greater than that a beam diameter of a transmitted white light by a high transmittance of the diaphragm for the infrared light, thus simultaneously ensuring a signal strength of the infrared light. Please refer to FIG. 5. The left graph is an experimental effect diagram for a fluorescence signal, when using the diaphragm with a high transmittance for fluorescence in this disclosure. Compared with the right graph using a traditional aperture stop, the signal intensity of the left graph is significantly higher than that of the right graph, by taking aperture stop as a single variable.

The above specific embodiments are configured to illustrate this disclosure, which are only configured to help understand this disclosure and are not intended to limit this disclosure. For those skilled in the art of the technical field to which this disclosure belongs, several simple deductions, deformations or substitutions can be made according to the idea of this disclosure.

## Claims

1. An endoscope, **characterized in that**, comprising:
a tube unit, which is inserted into a portion to be observed; and
an optical imaging system, which is arranged inside the tube unit, wherein the optical imaging system comprises a diaphragm;
wherein the diaphragm is provided with a through-hole at a middle of the diaphragm along a radial direction of the diaphragm, wherein the through-hole forms a first light-transmitting region; the diaphragm is further provided with a second light-transmitting region, which is located outside the first light-transmitting region along a radial direction of the first light-transmitting region; wherein the second light-transmitting region is configured to absorb a light within a first wavelength range and transmit a light within a second wavelength range; wherein the light within the first wavelength range is a light that is reflected after a light source of the endoscope irradiates the portion to be observed, and the light within the second wavelength range is fluorescence that is generated by a fluorescent dye at the portion to be observed when the light source of the endoscope irradiates the fluorescent dye.

2. The endoscope according to claim 1, **characterized in that**, the diaphragm is arranged at a position of an aperture stop of the optical imaging system, and/or at conjugate position(s) of object and image of an aperture stop of the optical imaging system.

3. The endoscope according to claim 2, **characterized in that**, the endoscope is a rigid endoscope, and the endoscope comprises a rod lens assembly for image inversion, wherein the rod lens assembly comprises a pair of rod lenses, and the diaphragm is arranged between the pair of rod lenses.

4. The endoscope according to claim 3, **characterized in that**, at least a portion of respective end surfaces of respective adjacent ends of the pair of rod lenses is a convex spherical surface; and at least a portion of the convex spherical surface is inserted into the through-hole at the middle of the diaphragm.

5. The endoscope according to claim 1, **characterized in that**, the endoscope is a flexible endoscope or an electronic endoscope, and the diaphragm is located on an objective lens of the flexible endoscope or the electronic endoscope.

6. An endoscope, **characterized in that**, comprising:
a tube unit, which is inserted into a portion to be observed; and
an optical imaging system, which is arranged inside the tube unit, wherein the optical imaging system comprises a diaphragm;
wherein the diaphragm is provided with a first light-transmitting region, which is located at a middle of the diaphragm along a radial direction of the diaphragm, and a second light-transmitting region, which is located outside the first light-transmitting region along a radial direction of the first light-transmitting region; wherein the first light-transmitting region is configured to transmit a light within a first wavelength range and a light within a second wavelength range; the second light-transmitting region is configured to absorb the light within the first wavelength range and transmit the light within the second wavelength range; wherein the light within the first wavelength range is a light that is reflected after a light source of the endoscope irradiates the portion to be observed, and the light within the second wavelength range is fluorescence that is generated by a fluorescent dye at the portion to be observed when the light source of the endoscope irradiates the fluorescent dye.

7. An endoscope, **characterized in that**, comprising:
a tube unit, which is inserted into a portion to be observed; and
an optical imaging system, which is arranged inside the tube unit, wherein the optical imaging system comprises a diaphragm;
wherein the diaphragm is provided with a through-hole at a middle of the diaphragm along a radial direction of the diaphragm, wherein the through-hole forms a first light-transmitting region; the diaphragm is further provided with a second light-transmitting region, which is located outside the first light-transmitting region along a radial direction of the first light-transmitting region; wherein the second light-transmitting region is configured to absorb a light within a first wavelength range and transmit a light within a second wavelength range; wherein a wavelength value of the first wavelength range is less than a wavelength value of the second wavelength range.

8. The endoscope according to any one of claims 1-7, **characterized in that**, a physical thickness d of the diaphragm is configured to be: d>0.3 mm.

9. The endoscope according to any one of claims 1-7, **characterized in that**, the light within the first wavelength range is a visible light, and a transmittance for the visible light of the second light-transmitting region is no more than 1%.

10. The endoscope according to any one of claims 1-7, **characterized in that**, the light within the second wavelength range is a near-infrared light, and a transmittance for the near-infrared light of the second light-transmitting region is not less than 50%.

11. The endoscope according to any one of claims 1-7, **characterized in that**, an outer diameter of the second light-transmitting region is greater than 1.2 times of an outer diameter of the first light-transmitting region.

12. The endoscope according to any one of claims 1-7, **characterized in that**, the first light-transmitting region is made of a homogeneous material, and/or the second light-transmitting region is made of a homogeneous material.

13. The endoscope according to claim 12, **characterized in that**, a material of the first light-transmitting region is one of: acrylonitrile-butadiene-styrene copolymer, polymethyl methacrylate, polyethylene terephthalate plastic, polyetherimide, and polycarbonate; and/or
a material of the second light-transmitting region is one of: acrylonitrile-butadiene-styrene copolymer, polymethyl methacrylate, polyethylene terephthalate plastic, polyetherimide, and polycarbonate.

14. The endoscope according to any one of claims 1-7, **characterized in that**, a high-temperature resistance of the diaphragm is not less than 110 degrees Celsius.

15. The endoscope according to any one of claims 1-7, **characterized in that**, the diaphragm is provided with a third light-transmitting region, which is located between the first light-transmitting region and the second light-transmitting region, and/or is located outside the second light-transmitting region along a radial direction of the second light-transmitting region.

16. A diaphragm for an optical imaging system of an endoscope, **characterized in that**, comprising:
a first light-transmitting region, which is located at a middle of the diaphragm along a radial direction of the diaphragm; and
a second light-transmitting region, which is located outside the first light-transmitting region along a radial direction of the first light-transmitting region;
wherein the first light-transmitting region is configured to transmit a light within a first wavelength range and a light within a second wavelength range; the second light-transmitting region is configured to absorb the light within the first wavelength range and transmit the light within the second wavelength range; wherein the light within the first wavelength range is a light that is reflected after a light source of the endoscope irradiates the portion to be observed, and the light within the second wavelength range is fluorescence that is generated by a fluorescent dye at the portion to be observed when the light source of the endoscope irradiates the fluorescent dye; wherein a wavelength value of the first wavelength range is less than a wavelength value of the second wavelength range.

17. The diaphragm according to claim 16, **characterized in that**, the diaphragm is provided with a through-hole at the middle of the diaphragm along the radial direction of the diaphragm, wherein the through-hole forms the first light-transmitting region.
